# EUROPEAN PATENT SPECIFICATION

(11) **EP 0 375 106 B1**
(45) Date of publication and mention of the grant of the patent: **04.01.1995**
(21) Application number: 89306836.1
(22) Date of filing: 05.07.1989
(51) Int. Cl.: A61B 5/00, A61B 5/0484

(54) **System for evoking electroencephalogram signals**
System zur Evozierung von elektroencephalographischen Signalen
Système pour évoquer des signaux électro-encéphalographiques

(30) Priority: 23.12.1988 JP 323698/88; 23.12.1988 JP 323699/88; 23.12.1988 JP 323700/88
(43) Date of publication of application: 27.06.1990
(73) Proprietor: PIONEER ELECTRONIC CORPORATION, Meguro-ku Tokyo (JP)
(72) Inventor: Yasushi, Mitsuo Kawagoe Works, Kawagoe-shi Saitama-ken (JP); Saito, Yoshio Kawagoe Works, Kawagoe-shi Saitama-ken (JP)
(74) Representative: Brunner, Michael John

(56) References cited:
- US-A- 3 032 029
- US-A- 3 753 433
- US-A- 3 942 516
- US-A- 4 031 883
- US-A- 4 334 545
- Proceedings of the IEEE NAECON 1988, May 23-27, 1988. vol. 4, pages 1523 -1529; JUNKER et al.: "Loop-closure of the visual-cortical response."
- Proceedings of the IEEE NAECON 1988, May 23-27, 1988. vol. 4, pages 1530 -1535; SCHNURER et al.: "A real time frequency analysis methodology for evoked potential loop-closure"

## Description

The present invention relates to a system for evoking a desired electroencephalogram signal, i.e., a brain wave, efficiently from a human brain.

It is known in the art that brain waves, i.e., electroencephalogram (EEG) signals, originating from the brain of a human being and the physiological and psychological states of the human being are closely related to each other. For example, when a human being is in a relaxed state, alpha (α) waves in a frequency range of from about 8 to 13 Hz are dominantly produced. Beta (β) waves are prevalent in an active mental state and have a frequency ranging from about 14 to 30 Hz. Brain waves generated during drowsiness and light sleep are theta (ϑ) waves in a frequency band ranging from about 4 to 7 Hz. The correlation between these brain waves having different frequency ranges and certain human activity phases indicates that the evocation of a certain brain wave through sensory stimulation is apt to put the human being in a corresponding physiological and psychological state.

Based on the analysis of the interaction between the EEG signals and physiological and psychological states, there have heretofore been proposed various systems for evoking alpha waves from the brain of a human being by giving a certain external stimulus to his body, to thereby place him in a physically and psychologically relaxed state for assisting him in lessening stresses and achieving mental concentration.

For example, Japanese Laid-Open Patent Publication No. 55-63656 discloses a biofeedback device for putting a user in a relaxed state by picking up brain waves through electrodes attached to the scalp of the user, extracting alpha waves from the brain waves, and varying sounds emitted from earphones worn by the user depending on the intensity of the alpha waves to let the user known how the alpha waves are being produced based on different sounds.

A relaxation device disclosed in Japanese Laid-Open Patent Publication No. 62-87165 includes a random noise generator for generating random noise to artificially produce an alpha wave signal having fluctuating 1/f characteristics. A light source is turned on and off by the alpha wave signal to apply a photic stimulus to a user for thereby placing the user in a relaxed state.

For the user to reach a relaxed state with the biofeedback device described above, it is necessary that the user differentiate differing sounds emitted from the earphones and control his psychological state according to his own will in order to make the sound corresponding to the alpha waves most intensive. Before the biofeedback device is used most effectively, therefore, the user has to repeatedly practice the device to find out how mental concentration should be effected to produce intensive alpha waves, so that the psychological state can freely be changed to some extent according to the user's own will. A person who uses the biofeedback device may not necessarily achieve the desired result, and hence how the device turns out to be effective may vary from user to user.

The relaxation device disclosed in the latter publication employs an artificial alpha wave signal which has no direct relationship whatsoever to the brain waves of a user for evoking the brain waves. Accordingly, the evoked EEG responses are not constant and tend to differ from one user to another.

In view of the aforesaid drawbacks of the conventional electroencephalogram evoking systems, it is an object of the present invention to provide an electroencephalogram signal evoking system for quickly evoking a desired brain wave or electroencephalogram signal such as an alpha, beta, or theta wave, without the need for any preliminary training or practice on the part of a user of the system, by extracting only a signal corresponding to the desired brain wave from signals indicating physiological changes of the system user and feeding back the extracted signal as a stimulating signal to the system user. US-A-4031883 discloses a biofeedback system for training a user to control his electroencephalogram signals. The proceedings of the IEEE NAECON 1988, 23-27 May, Vol. 4, pages 1523-1535, over which the present invention is characterised, discloses an EEG feedback system and frequency analysis system for EEG signals.

According to the present invention, there is provided a system for evoking an electroencephalogram signal from the brain of a user of the system, comprising
detecting means for detecting a physiological change in a body of the user and producing a signal indicative of the detected physiological change;
an amplifier connected to the detecting means for amplifying the signal indicative of the detected physiological change and producing an amplified signal;
filtering means for filtering the amplified signal; and
stimulating means for converting the frequency signal extracted by the filtering means to a stimulative signal and for applying the stimulative signal to a user; characterised by:
the filtering means extracting an electroencephalogram signal to be evoked from the signal produced by the detecting means; and
the stimulating means generating a stimulative signal at a frequency which is in synchronism with the signal extracted by the filtering means.

The above and other objects, features and advantages of the present invention will become more apparent from the following description when taken in conjunction with the accompanying drawings in which preferred embodiments of the present invention are shown by way of illustrative example.
Fig. 1 is a block diagram of a system for evoking an electroencephalogram signal according to an embodiment of the present invention;
Fig. 2 is a diagram showing a measured alpha wave evoked by the EEC signal evoking system shown in Fig. 1;
Fig. 3 is a block diagram of a system for evoking an electroencephalogram signal according to another embodiment of the present invention;
Fig. 4 is a block diagram of a filter frequency control circuit in the EEC signal evoking system illustrated in Fig. 3;
Figs. 5(a) and 5(b) are diagrams showing the spectrums of alpha waves before and during photic stimulation in the EEG signal evoking system of Fig. 3;
Fig. 6 is a block diagram of a system for evoking an electroencephalogram signal according to still another embodiment of the present invention;
Fig. 7 is a diagram showing a measured alpha wave evoked when a photic stimulus signal of an adequate level is applied;
Fig. 8 is a diagram showing a measured alpha wave evoked when a photic stimulus signal that is too intensive is applied;
Fig. 9 is a block diagram of a system for evoking an electroencephalogram signal according to yet another embodiment of the present invention;
Fig. 10 is a block diagram of a system for evoking an electroencephalogram signal in accordance with a further embodiment of the present invention;
Fig. 11 is a diagram illustrating the waveforms of signals produced in the EEG signal evoking system shown in Fig. 10;
Fig. 12 is a block diagram of a system for evoking an electroencephalogram signal in accordance with a still further embodiment of the present invention;
Fig. 13 is a block diagram of a pulse width modulator in the EEG signal evoking system shown in Fig. 12;
Fig. 14 is a diagram showing the waveforms of signals produced in the EEG signal evoking system shown in Fig. 12;
Fig. 15 is a block diagram of a system for evoking an electroencephalogram signal according to a yet further embodiment of the present invention;
Fig. 16 is a diagram showing the principles of operation of the EEG signal evoking system shown in Fig. 15; and
Fig. 17 is a block diagram similar to Fig. 15, showing an EEG signal evoking system according to another embodiment of the present invention.

Like or corresponding parts are denoted by like or corresponding reference characters throughout views.

Brain waves or EEG signals of a human being tend to be synchronized by external stimulative signals applied to the body of the human being. The principles of the present invention are based on such synchronization of brain waves.

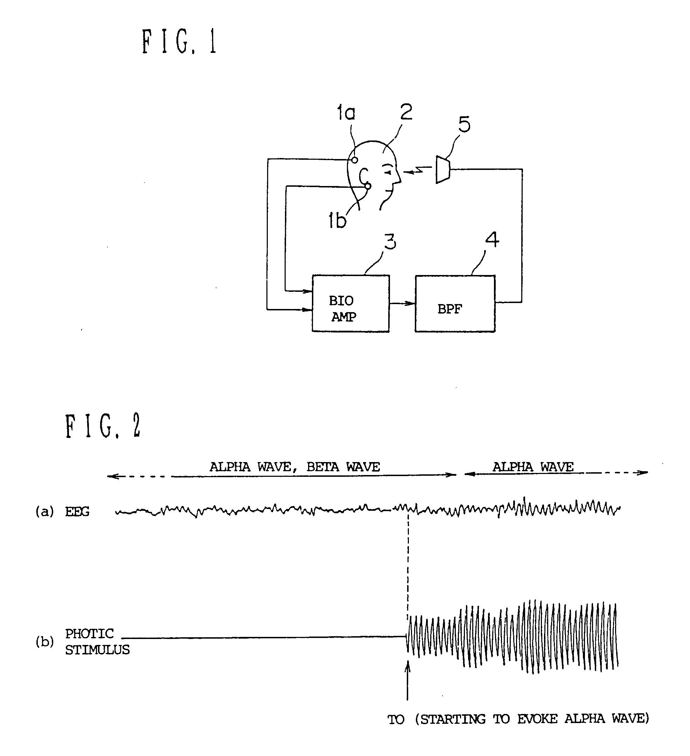

Fig. 1 shows in block form a system for evoking an electroencephalogram (EEG) signal or a brain wave in accordance with the present invention. The EEG signal evoking system shown in Fig. 1 employs an EEG signal as indicating a physiological change detected from the body of a system user, and also employs a photic stimulus as a stimulating signal to be applied to the user's body.

The EEG signal evoking system includes a pair of electrodes 1a, 1b for picking up EEG signals or brain waves from the scalp 2 of the system user, a biological signal amplifier 3 for amplifying the EEG signals picked up by the electrodes 1a, 1b to a signal level suitable for subsequent signal processing, a bandpass filter 4 coupled to the biological signal amplifier 3, and a photic stimulus generator 5 connected to the bandpass filter 4.

The bandpass filter 4 passes only a signal corresponding to a brain wave desired to be evoked. If an alpha wave having a frequency ranging from 8 to 13 Hz is to be evoked, then the central frequency of the passband of the bandpass filter 4 is selected to be f₀ = 10 Hz and sharpness Q of the bandpass filter 4 is selected to be Q = 10. If a theta wave having a frequency range of from 4 to 7 Hz is to be evoked, then the central frequency is selected to be f₀ = 5 Hz and the Q is selected to be Q = 10. If a beta wave having a frequency range of from 14 to 30 Hz is to be evoked, the central frequency is selected to be f₀ = 20 Hz and the Q is selected to be Q = 4.

The photic stimulus generator 5 generates stimulating light which flickers in synchronism with changes in the amplitude of a signal produced by the bandpass filter 4. The photic stimulus generator 5 may comprise a light-emitting device such as an LED and a driver for energizing the light-emitting device.

The electrodes 1a, 1b and the photic signal generator 5 are incorporated in a headband (not shown), for example. When the headband is worn on the user's scalp 2, the electrodes 1a, 1b contacts the scalp 2 at prescribed positions thereon, and the light-emitting device of the photic stimulus generator 5 is positioned in front of an eye of the user. Therefore, brain waves can be picked up from the scalp 2 and a photic stimulus can be applied to the user quite easily at the same time.

Operation of the EEG signal evoking system for evoking an alpha wave, for example, will be described below. For alpha wave evocation, the central frequency of the pass band of the bandpass filter 4 is set f₀ = 10 Hz and the Q thereof is set to Q = 10, as described above.

The user wears the headband on the scalp 2 and the EEG signal evoking system is switched on. The electrodes 1a, 1b pick up EEG signals or brain waves from the scalp 2 and send them to the biological signal amplifier 3 which amplifies the EEG signals. The amplified EEG signals are then fed to the bandpass filter 4 which selects and passes only a signal having a frequency around 10 Hz corresponding to that of the desired alpha wave. The signal from the bandpass filter 4 is then applied to the photic stimulus generator 5.

The photic stimulus generator 5 is responsive to the alpha wave signal sent from the bandpass filter 4 for turning on and off the light-emitting device such as an LED to radiate flickering light synchronous with the alpha wave as a photic stimulus toward the user.

When the flickering photic stimulus is applied or fed back to the user for visual stimulation, the alpha wave of the brain waves is synchronized by the flickering light and is strongly evoked. The evoked alpha wave is then picked up by the electrodes 1a, 1b and sent to the biological signal amplifier 3, after which the above process is repeated.

Therefore, when the evocation of the alpha wave is started, a closed loop is established by the EEG signal evoking system and the user, thereby providing an oscillator which oscillates at the frequency of the alpha wave signal that has been selected by the bandpass filter 4. Only a signal corresponding to the alpha wave to be evoked circulates through the closed loop, so that only the desired alpha wave is strongly evoked, rapidly bringing the user into a desired evoked EEG condition. As a result, the alpha wave of the brain waves becomes quickly intensive, and hence the percentage of the alpha wave in the brain waves and the peak value of the alpha wave are appreciably increased.

Fig. 2 shows the measured waveform of an alpha wave evoked by the EEG signal evoked system shown in Fig. 1. Brain waves picked up by the electrodes 1a, 1b are indicated at (a) in Fig. 2, whereas a photic stimulus signal applied to the user by the photic stimulus generator 5 is indicated at (b) in Fig. 2. The EEG signal evoking system is switched on at a time t₀ to start evoking the alpha wave.

It is apparent from Fig. 2 that before the time t when no alpha wave is evoked, brain waves including various waves such as alpha, beta, and other waves are present. When the EEG signal evoking system is turned on to start to evoke the alpha wave, the alpha wave quickly becomes prevalent. Therefore, the EEG signal evoking system is highly effective in quickly putting the user in a relaxed state. Since a signal indicating a physiological change in the user's body is used as a stimulative signal applied to the user's body, the EEG signal evoking system of the invention well matches the user himself and is highly effective to evoke the desired brain wave.

In the above embodiment, although the biological signal amplifier 3 has been described as being a flat amplifier, the biological signal amplifier 3 may be imposed with a filtering characteristic. Specifically, in order to evoke thetha wave having a frequency ranging from 0.5 to 7 Hz, the biological signal amplifier 3 may have a filtering characteristic such as to pass the EEG signal having a frequency ranging from 0.5 to 20 Hz. In this case, the bandpass filter 4 can be replaced with a low-pass filter allowing to pass the EEG signal having a frequency lower than 7 Hz. It would be apparent that a high-pass filter can also be employed in lieu of the bandpass filter 4 depending upon the filtering characteristic imposed to the biological signal amplifier.

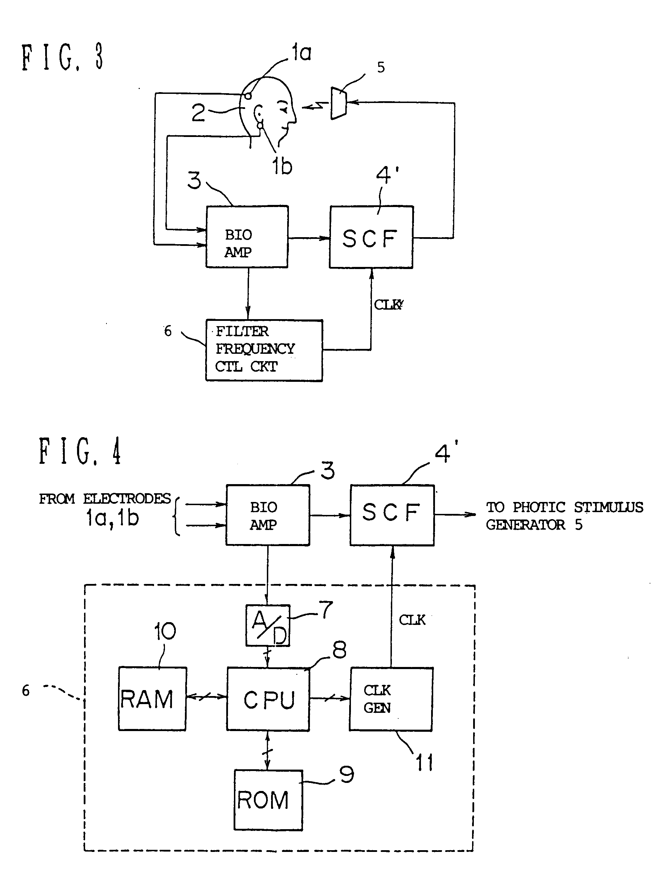

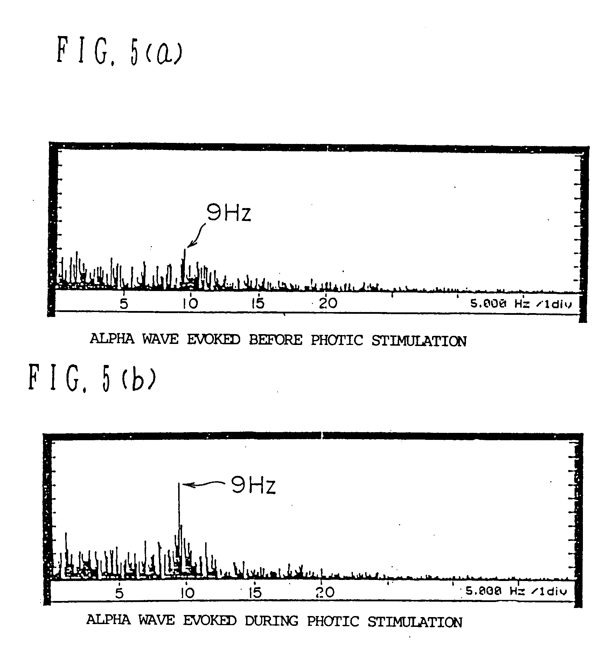

Figs. 3 and 4 show an EEG signal evoking system according to another embodiment of the present invention. The EEG signal evoking system of Figs. 3 and 4 differs from the EEG signal evoking system shown in Fig. 1 in that a switched capacitor filter (SCF) 4′ is used as a bandpass filter connected to the biological signal amplifier 3, and a filter frequency control circuit 6 is employed to vary the central frequency f₀ of the passband of the SCF 4′. Those components of the EEG signal evoking system which are identical to those shown in Fig. 1 will not be described in detail.

The SCF 4′ is of a known construction and can freely vary the central frequency f of the passband thereof depending on the frequency of a clock signal applied thereto.

As shown in Fig. 4, the filter frequency control circuit 6 comprises an analog-to-digital (A/D) converter 7, a central processing unit (CPU) 8, a read-only memory (ROM) 9, a random-access memory (RAM) 10, and a clock generator 11. The filter frequency control circuit 6 analyzes the frequencies of the brain waves or EEG signals from the biological signal amplifier 3 through fast Fourier transform (FFT) or autocorrelation to produce a brain wave frequency spectrum, selects from the brain wave frequency spectrum a frequency that is most suitable for evoking a desired brain wave, and sets the central frequency f₀ of the passband of the SCF 4′ to the selected frequency.

The frequencies of alpha waves produced by different users vary from one user to another and range from 8 to 13 Hz. If the photic stimulus signal has a fixed frequency of 10 Hz, for example, for evoking an alpha wave with its frequency ranging from 8 to 13 Hz, then alpha waves cannot equally effectively be evoked from different users. The EEG signal evoking system shown in Figs. 3 and 4 is designed to solve this drawback.

In operation, the amplified brain waves or EEG signals are fed from the biological signal amplifier 3 to the SCF 4′ and the filter frequency control circuit 6.

The brain waves delivered to the filter frequency control circuit 6 are converted by the A/D converter 7 to digital signals which are then applied to the CPU 8. The CPU 8 converts the time-based digital brain signal from the A/D converter 7 to a frequency-base brain wave spectrum by way of fast Fourier transform, and selects from the brain wave spectrum a frequency which is best suited to evoke a desired brain wave. The CPU 8 controls the frequency of the clock signal generated by the clock generator 11 in order to equalize the central frequency f₀ of the passband of the SCF 4′ to the selected frequency. The clock generator 11 then supplies the clock signal with the controlled frequency to the SCF 4′. As a consequence, the central frequency f₀ of the passband of the SCF 4′ is set to the optimum brain wave evoking frequency. The SCF 4′ then only passes a signal present in the passband thereof having the central frequency f0, and the signal from the SCF 4′ is fed to the photic stimulus generator 5.

Accordingly, only the signal having the optimum frequency that has been selected by the filter frequency control circuit 5 circulates through the closed loop composed of the EEG signal evoking system and the user. An alpha wave, for example, is synchronized by the optimum frequency, and quickly and strongly evoked.

Fig. 5(a) shows a brain wave spectrum produced before a photic stimulus is applied to the user by the EEG signal evoking system of Figs. 3 and 4, and Fig. 5(b) shows a brain wave spectrum produced while a photic stimulus is being applied to the user by the EEG signal evoking system shown in Figs. 3 and 4.

A signal having a frequency of 9 Hz has a maximum spectral intensity in the alpha-wave frequency range of from 8 to 13 Hz in the brain wave spectrum produced before photic stimulation shown in Fig. 5(a). The frequency of 9 Hz is selected as the optimum brain wave evoking frequency by the filter frequency control circuit 6, which then automatically sets the central frequency f₀ of the SCF 4′ to 9 Hz. Comparison between Figs. 5(a) and 5(b) clearly indicates that the alpha wave having the frequency of 9 Hz is selectively evoked highly intensively by applying the photic stimulus to the user with the EEG signal evoking system shown in Figs. 3 and 4.

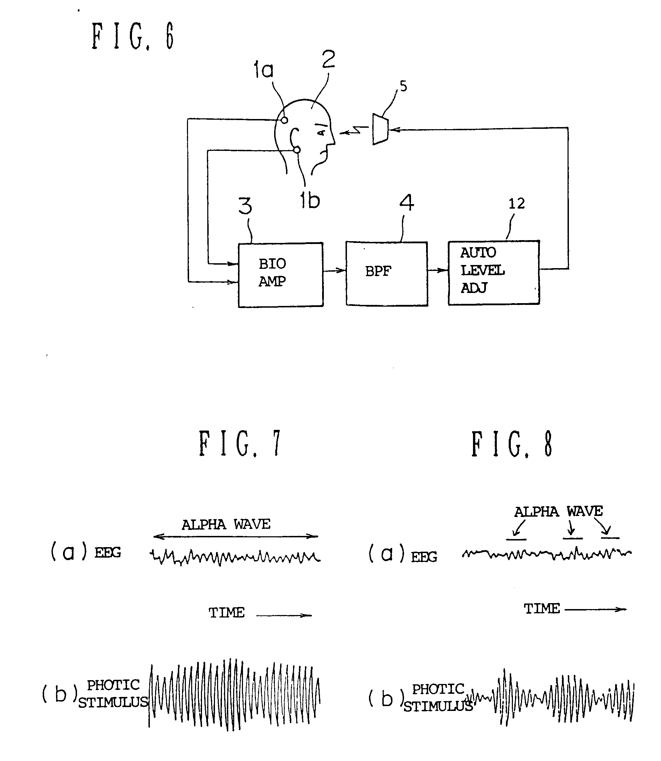

Fig. 6 shows an EEG signal evoking system according to still another embodiment of the present invention. The EEG signal evoking system shown in Fig. 6 is similar to the EEG signal evoking system shown in Fig. 1 except that an automatic level adjusting circuit 12 is connected between the bandpass filter 4 and the photic stimulus generator 5. Those parts of the EEG signal evoking system which are identical to those shown in Fig. 1 will not be described in detail.

The automatic level adjusting circuit 12 comprises, for example, an automatic gain control (AGC) circuit for keeping a signal level at a constant level at all times, or a limiter or a clipper for holding the maximum amplitude of a signal below a certain level. An output signal from the automatic level adjusting circuit 12 is applied to the photic stimulus generator 5.

If a photic stimulus applied to the user were too weak, it would fail to synchronize a desired brain wave intensively enough, and if the photic stimulus applied to the user were too strong, its synchronizing effect would automatically be suppressed owing to the physiological and psychological protective reactions of the user's body. Unless the brain wave is evoked by a stimulative signal having an appropriate level, the amplitude of the evoked brain wave would periodically be increased and reduced, and the desired brain wave could not efficiently be evoked. The EEG signal evoking system shown in Fig. 6 is designed to avoid this shortcoming and evoke a desired brain wave efficiently.

The EEG signal evoking system shown in Fig. 6 operates as follows: The signal from the bandpass filter 4, which has a frequency near 10 Hz corresponding to that of a desired alpha wave, is applied to the automatic level adjusting circuit 12. The automatic level adjusting circuit 12 then adjusts the signal from the bandpass filter 4 to a predetermined level, and sends the alpha-wave signal with the adjusted level to the photic stimulus generator 5.

Fig. 7 shows the measured waveform of an alpha wave which is evoked by the EEG signal evoking system when a photic stimulus signal is generated at a adequate level. Fig. 8 illustrates the measured waveform of an alpha wave which is evoked when no level adjustment is effected on the signal from the bandpass filter 4 and a photic stimulus signal is too intensive.

If the photic stimulation applied to the user is too strong as shown at (b) in Fig. 8, then the amplitude of an evoked alpha wave tends to vary periodically due to physiological and psychological protective reactions of the user. Therefore, the evoked alpha wave is intermittently produced as shown at (a) in Fig. 8. If the photic stimulation is of an adequate level as shown at (b) in Fig. 7, then the evoked alpha wave is continuously produced as shown at (a) in Fig. 7.

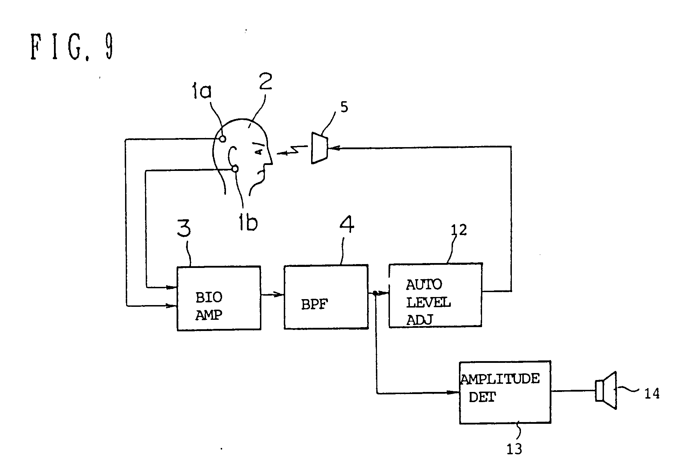

According to yet another embodiment shown in Fig. 9, an EEG signal evoking system includes, in addition to the system components shown in Fig. 6, an amplitude detector 13 connected to the output terminal of the bandpass filter 4 and an audible sound generator 14 such as a loudspeaker, an earphone, an electronic buzzer, or the like.

Brain waves or EEG signals are usually evoked from the brain of the user while the eyes of the user are being fully or partly closed. Therefore, foe efficient brain wave evocation, it is preferable to let the user know aurally the level of the evoked brain wave to allow the user to confirm the condition in which the brain wave is evoked while the brain wave is being evoked. The EEG signal evoking system illustrated in Fig. 9 permits the user to confirm aurally the level of the evoked brain wave.

The amplitude detector 7 monitors the amplitude level of a brain wave signal produced by the bandpass filter 4 and detects the level of the brain wave evoked at the time depending on the magnitude of the detected amplitude level. The amplitude detector 7 then applies an audio signal commensurate with the level of the evoked brain wave to the audible sound generator 14.

When an alpha wave starts being evoked by the EEG signal evoking system, the amplitude detector 7 monitors the alpha wave signal generated by the bandpass filter 4 and detects the level of the evoked alpha wave at the time based on the magnitude detector 7 generates an audio signal depending on the magnitude of the level of the evoked alpha wave, and applies the audio signal to the audible sound generator 14. The audible sound generator 14 then generates a sound to let the user know aurally how the desired alpha wave is evoked. The user can thus confirm the condition in which the alpha wave is evoked while the alpha wave is being evoked.

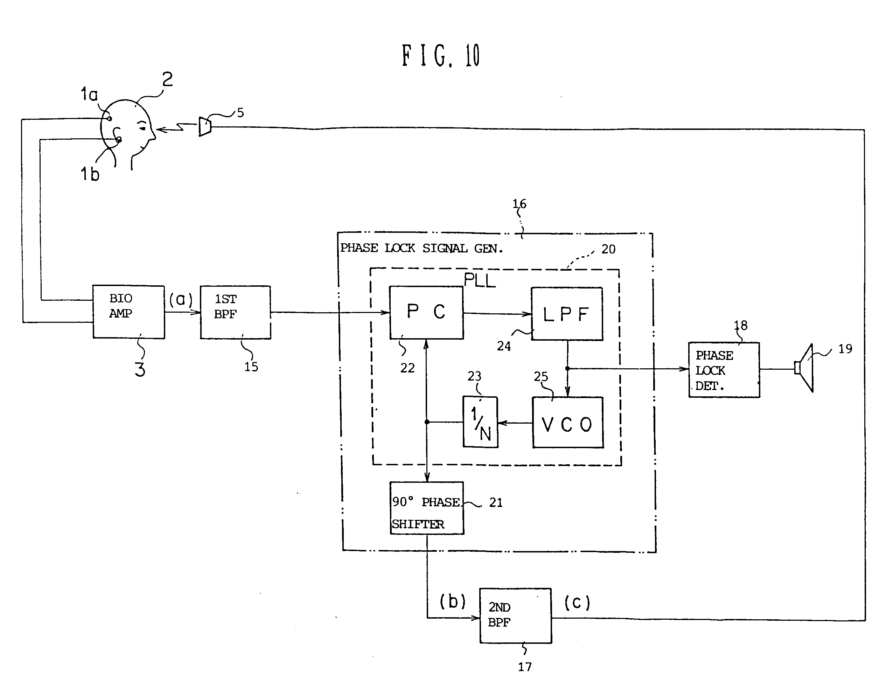

Fig. 10 shows in block form a system for evoking an electroencephalogram signal according to a further embodiment of the present invention. The EEG signal evoking system shown in Fig. 10 has the electrodes 1a, 1b, the biological signal amplifier 3, and the photic stimulus generator 5 which are identical to those of the EEG signal evoking system shown in Fig. 1. The EEG signal evoking system of Fig. 10 additionally includes a first bandpass filter 15 for removing noise from brain wave signals sent from the biological signal amplifier 3, a phase lock signal generator 16 for generating a rectangular wave signal of a constant level in phase with the brain wave signal from the first bandpass filter 15, a second bandpass filter 17 for extracting only a certain frequency signal corresponding to a brain wave to be evoked, from the rectangular wave signal produced by the phase lock signal generator 16 and passing only the extracted frequency signal, a phase lock detector 18 for monitoring the phase locked condition of the phase lock signal generator 16 to detect brain wave fluctuations, and an audible sound generator 19 such as a loudspeaker, an earphone, an electronic buzzer, or the like for informing the user of the detected brain wave fluctuations as an audible sound. The output signal from the second bandpass filter 17 is applied to the photic stimulus generator 5 which generates and gives a correponding photic stimulus to the user.

The phase lock signal generator 16 serves to keep an evoked brain wave in phase with a stimulative signal applied or fed back to the user. Synchronization of the evoked brain wave by the stimulative signal becomes intensive and the brain wave is highly effective evoked when the evoked brain wave is kept in phase with the stimulative signal. The EEG signal evoking system shown in Fig. 10 is designed to effect such high brain wave evocation.

Each of the first and second bandpass filters 15, 17 serves to selectively pass a frequency signal corresponding to a brain wave to be evoked. The passband of each of the first and second bandpass filters 15, 17 is selected to range from 8 to 13 Hz if an alpha wave is to be evoked, from 4 to 7 Hz if a beta wave is to be evoked, and from 14 to 30 Hz if a theta wave is to be evoked. Since the first bandpass filter 15 primarily serves to remove noise from the brain waves, the passband of the first bandpass filter 15 may be wider than that of the second bandpass filter 17, or the first bandpass filter 15 may be dispensed with in some applications.

The phase lock signal generator 16 comprises a phase-locked loop (PLL) 20 for generating a square wave signal having the same frequency as that of an input signal applied from the first bandpass filter 15 to the PLL 20, and a 90° phase shifter 21 for shifting the phase of the square wave signal generated by the PLL 20. The PLL 20 comprises a phase comparator (PC) 22, a 1/N frequency divider 23, a low-pass filter (LPF) 24, and a voltage-controlled oscillator (VCO) 25.

In operation, noise except an alpha wave signal is removed by the first bandpass filter 15 from the brain waves or EEG signals amplified by the biological signal amplifier 3, and the alpha wave signal is then supplied to the phase lock signal generator 16.

The alpha wave signal from the first bandpass filter 15 is applied to the PC 22 in which it is compared with a frequency-divided signal from the 1/N frequency divider 23. The PC 22 then issues a voltage proportional to the phase difference between the alpha wave signal and the frequency-divided signal. The voltage is then converted by the LPF 24 to a DC voltage which is applied to the voltage-controlled oscillator 25.

In response to the applied DC voltage, the voltage-controlled oscillator 25 generates a square wave signal having a frequency which is N times the frequency of the alpha wave signal applied to the phase comparator 22. The square wave signal is then applied from the voltage-controlled oscillator 25 to the 1/N frequency divider 23 which frequency-divides the square wave signal by N. Therefore, the 1/N frequency divider 23 issues a square wave signal having the same frequency as that of the alpha wave.

Because of the operational characteristics of the PLL 20, the wave signal issued by the 1/N frequency divider 13 leads the input signal applied to the phase comparator 12, i.e., the alpha wave signal, by 90°. Thus, the square wave signal from the 1/N frequency divider 23 is applied to the 90° phase shifter 21, so that the phase of the square wave signal is delayed 90 ° thereby. The square wave signal produced from the 90° phase shifter 21 has a constant level and is in phase with the alpha wave signal.

The second bandpass filter 17 extracts, from the square wave signal from the 90° phase shifter 21, a frequency signal having a frequency ranging from 8 to 13 Hz and hence corresponding to an alpha wave to be evoked, and passes the extracted frequency signal to the photic stimulus generator 5.

Since the alpha wave signal circulating through the loop composed of the EEG signal evoking system and the user is locked in phase with the alpha wave produced by the user and picked up by the electrodes 1a, 1b, the stimulating light which is fed back to the user by the photic stimulus generator 5 flickers in phase with the alpha wave of the user. Consequently, the desired alpha wave is intensively and quickly evoked because it is strongly synchronized by the photic stimulus signal.

The phase lock detector 18 monitors the DC voltage produced by the LPF 24 for variations. Any fluctuations in the frequency of the brain wave picked up by the electrodes 1a, 1b are detected based on the variations in the DC voltage. Such detected brain wave fluctuations are aurally notified to the user through the audible sound generator 19. Consequently, the desired brain wae can be evoked while the user is aurally confirming the condition in which it is evoked.

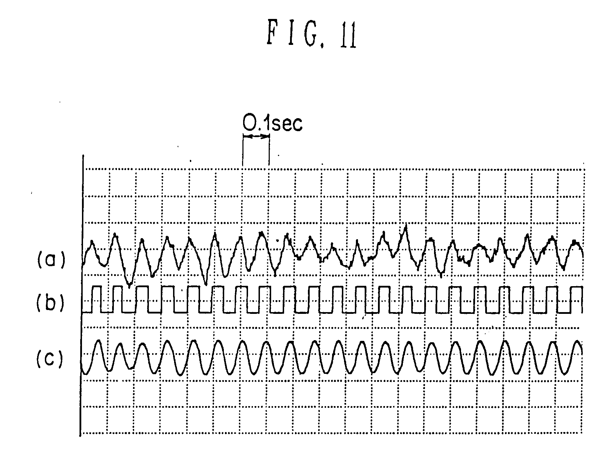

Fig. 11 illustrates the waveforms (a), (b), (c) of signals produced at corresponding points (a), (b), (c) in the EEG signal evoking system. Study of Fig. 11 clearly shows that the alpha wave picked up by the electrodes 1a, 1b, the square wave signal produced by the phase lock signal generator 20, and the output signal from the second bandpass filter 17 are in phase with each other. It will therefore be understood that the alpha wave is strongly synchronized by the photic stimulus signal produced by the photic stimulus generator 5.

The photic signal and the evoked alpha wave signal may be brought into phase with each other by a suitable phase correcting or shifting arrangement in the bandpass filter 4, the automatic level adjusting circuit 12, or the photic stimulus generator 5 in Fig. 9, rather than by the phase lock signal generator 16 shown in Fig. 10.

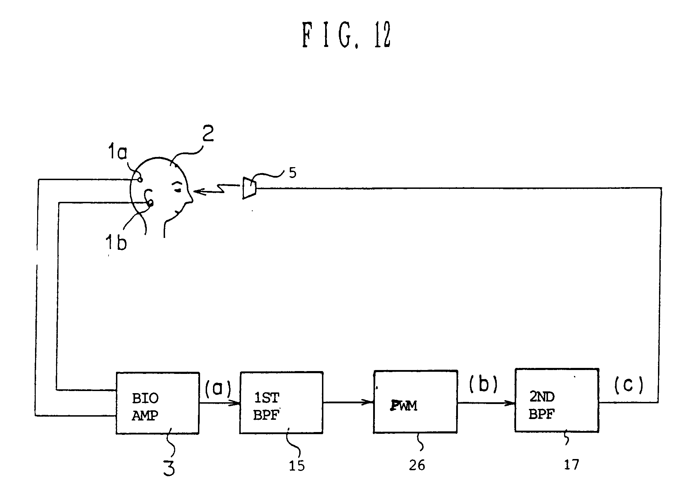

Fig. 12 shows a system for evoking an electroencephalogram signal according to a still further embodiment of the present invention. The EEG signal evoking system shown in Fig. 12 differs from the EEG signal evoking system illustrated in Fig. 10 only in that a pulse width modulator 26 is employed in place of the phase lock signal generator 16 in Fig. 10. Those parts which are identical to those shown in Fig. 10 will not be described in detail.

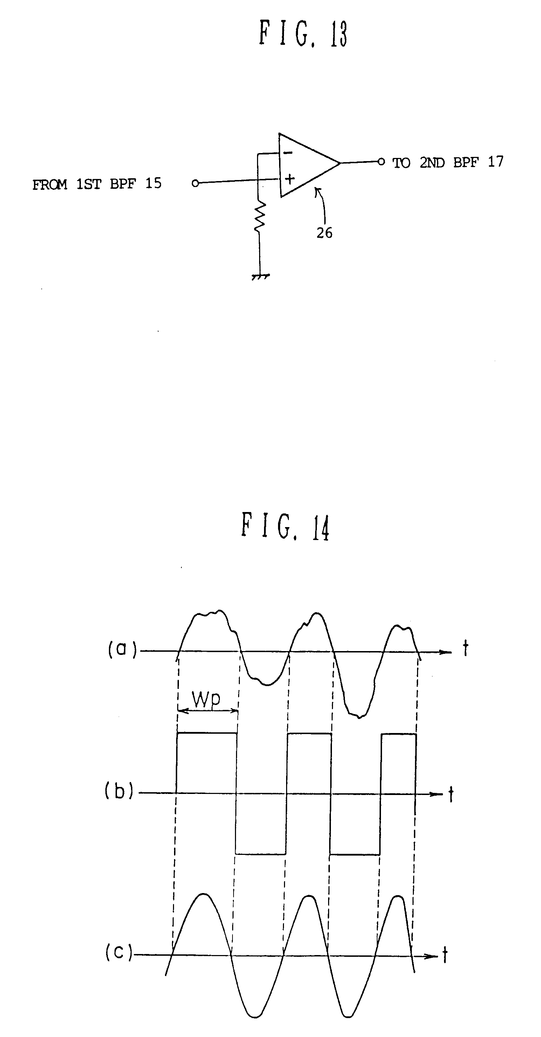

The pulse width modulator 26 serves to generate a square wave signal having a constant level which has been pulse-width-modulated by the brain wave signal applied from the first bandpass filter 15. The pulse width modulator 26 may comprise a known comparator, for example, as shown in Fig. 13.

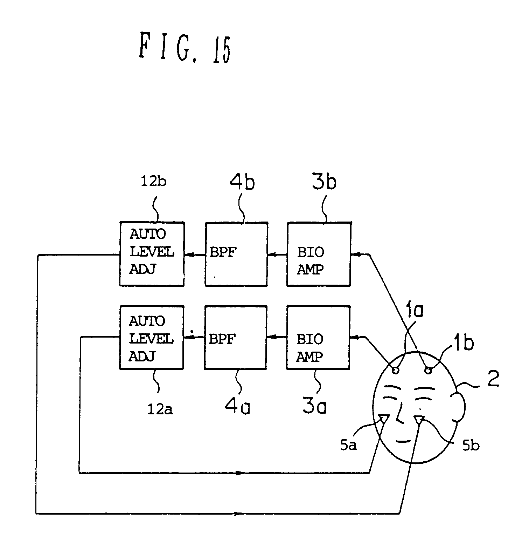

The EEG signal evoking system shown in Fig. 12 operates in the following manner: A brain wave signal picked up by the electrodes 1a, 1b is amplified by the biological signal amplifier 3 into a signal (shown at (a) in Fig. 14), and then a frequency signal corresponding to an alpha wave is selected from the amplified signal by the first bandpass filter 15 and applied to the pulse width modulator 26.

The pulse width modulator 26 generates a square wave signal which has been pulse-width-modulated by the alpha wave signal from the first bandpass filter 15, the square wave signal having a constant level and a pulse width Wₚ that varies depending on the alpha wave signal, as shown at (b) in Fig. 14. The square wave signal generated by the pulse width modulator 26 is therefore substantially in phase with the alpha wave signal applied to the pulse width modulator 26.

The second bandpass filter 17 extracts, from the square wave signal from the pulse width modulator 26, a frequency signal having a frequency ranging from 8 to 13 Hz and hence corresponding to an alpha wave to be evoked, and passes the extracted frequency signal to the photic stimulus generator 5.

As with the EEG signal evoking system shown in Fig. 10, the alpha wave picked up by the electrodes 1a, 1b, the square wave signal produced by the pulse width modulator 26, and the output signal from the second bandpass filter 17 are in phase with each other, as shown in Fig. 11. Therefore, the alpha wave is strongly sychronized by the photic stimulus signal produced by the photic stimulus generator 5.

Fig. 15 shows a system for evoking an electroencephalogram signal according to a yet further embodiment of the present invention.

Cerebrum physiology shows that the right and left cerebral hemispheres of the brain of a human being perform different functions. The left cerebral hemisphere chiefly governs the physiological and physical activities of the righthand half of the body, whereas the right cerebral hemisphere mainly controls the physiological and physical activities of the lefthand half of the body. Even when one of the cerebral hemispheres is placed in a brain wave evoking condition, the other cerebral hemisphere may not necessarily be put in the same brain wave evoking condition.

Generally, brain waves can most effectively be evoked from the right and left cerebral hemispheres when the brain waves are evoked synchronously. Desired brain waves can thus be evoked more effectively not by employing a signal indicating a physiological change in the body of the user as a stimulative signal, but also by stimulating the right cerebral hemisphere with a stimulative signal originating from the left cerebral hemisphere and stimulating the left cerebral hemisphere with a stimulative signal originating from the right cerebral hemisphere so that the right and left cerebral hemispheres will be stimulated by each other's brain waves. The EEG signal evoking system shown in Fig. 15 is arranged to evoke brain waves by feeding stimulative signals back to the right and left cerebral hemispheres.

The EEG signal evoking system shown in Fig. 15 includes a pair of electrodes 1a, 1b attached respectively to right and left portions of the forehead of the scalp 2 of a user to pick up brain waves or EEG signals, a pair of biological signal amplifiers 3a, 3b for amplifying the EEG signals to signal levels suitable for subsequent signal processing, a pair of bandpass filters 4a, 4b connected to the respective biological signal amplifiers 3a, 3b, a pair of automatic level adjusting circuits 12a, 12b connected respectively to the bandpass filters 4a, 4b, and a pair of photic stimulus generators 5a, 5b.

Each of the biological signal amplifiers 3a, 3b is identical to the biological signal amplifier 3 shown in Fig. 1. Each of the bandpass filters 4a, 4b is identical to the bandpass filter 4 shown in Fig. 1. Each of the automatic level adjusting circuits 12a, 12b is identical to the automatic level adjusting circuit 12 shown in Fig. 6. Each of the photic stimulus generators 5a, 5b is identical to the photic stimulus generator 5 illustrated in Fig. 1.
Therefore, the biological signal amplifiers 3a, 3b, the bandpass filters 4a, 4b, the automatic level adjusting circuits 12a, 12b, and the photic stimulus generators 5a, 5b will now be described in detail below.

The electrodes 1a, 1b and the photic stimulus generators 5a, 5b are mounted on a headband, for example, such that when the user wear the headband on the scalp 2, the electrodes 1a, 1b will be held respectively against the right and left portions of the forehead and the photic stimulus generators 5a, 5b will be positioned in front of the right and left eyes, respectively, of the user.

Operation of the EEG signal evoking system for evoking an alpha wave, for example, will be described below. For alpha wave evocation, the central frequency of the pass band of each of the bandpass filters 4a, 4b is set f₀ = 10 Hz and the Q thereof is set to Q = 10.

The user wears the headband on the scalp 2 and the EEG signal evoking system is switched on. The electrodes 1a, 1b pick up EEG signals or brain waves originating from the right and left cerebral hemispheres of the brain, and send them to the respective biological signal amplifiers 3a, 3b which amplify the EEG signals. The amplified EEG signals are then fed to the bandpass filters 4a, 4b which each select and pass only a signal having a frequency around 10 Hz corresponding to that of the desired alpha wave. The signals from the bandpass filters 4a, 4b are then applied to the automatic level adjusting circuits 12a, 12b, respectively, which adjust the signals to a predetermined level. The level-adjusted signals are then delivered to the photic stimulus generators 5a, 5b.

The photic stimulus generators 5a, 5b are responsive to the alpha wave signals sent from th automatic level adjusting circuits 12a, 12b for turning on and off the light-emitting devices such as LEDs with the alpha wave signals originating from the right and left cerebral hemispheres. The light-emitting devices radiate flickering light synchronous with these alpha wave signals as photic stimuli to the right and left eyes, respectively, of the user in order to evoke desired alpha waves.

The alpha waves evoked from the right and left cerebral hemispheres are picked up again by the respective electrodes 1a, 1b and sent to the biological signal amplifiers 3a, 3b, after which the above process is repeated.

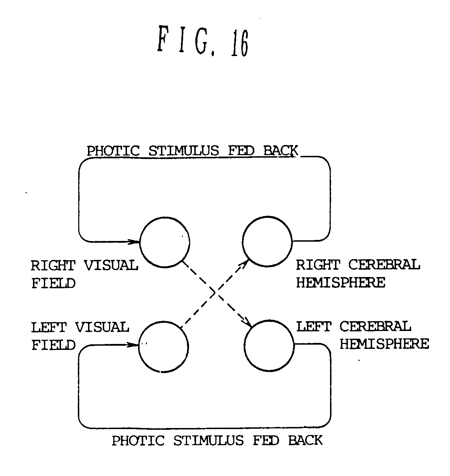

As shown in Fig. 16, the right and left cerebral hemispheres of a brain are linked to left and right visual fields by a nervous system. More specifically, the optic nerve for carrying sensation of sight from the right visual field of the right eye is connected to the left cerebral hemisphere, and the optic nerve for carrying sensation of sight from the left visual field of a left eye is connected to the right cerebral hemisphere. Therefore, a photic stimulus applied to the right eye stimulates the left cerebral hemisphere, and a photic stimulus applied to the left eye stimulates the right cerebral hemisphere. When the photic stimuli in the form of flickering light are applied by the photic stimulus generators 5a, 5b to the right and left eyes of the user, the photic stimulus produced by the alpha wave originating from the right cerebral hemisphere is applied through the right visual field to the left cerebral hemisphere to evoke an alpha wave therefrom, and the photic stimulus produced by the alpha wave originating from the left cerebral hemisphere is applied through the left visual field to the right cerebral hemisphere to evoke an alpha wave therefrom.

Consequently, when the evocation of the alpha waves is started, a closed loop is established by the EEG signal evoking system and the user, thereby providing an oscillator including the left and right cerebral hemispheres in series with each other, as shown in Fig. 2. Only signals indicating alpha waves that are to be evoked circulate through the closed loop which extends from the right cerebral hemisphere to the right visual field to the left cerebral hemisphere to the left visual field. As a result, desired alpha waves are intensively and quickly evoked from the right and left cerebral hemispheres of the brain of the user by being synchronized by the signals circulating through the closed loop.

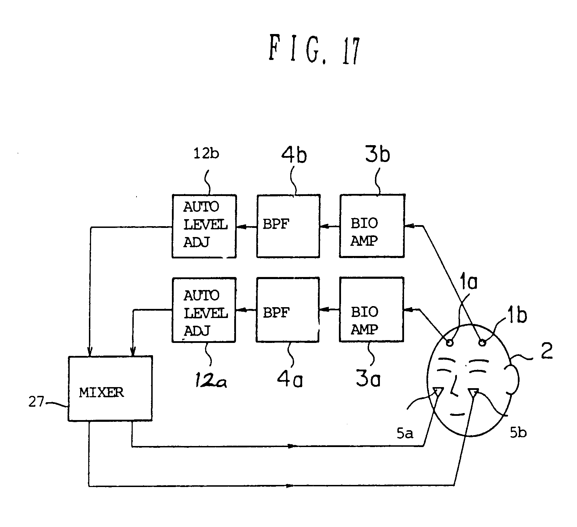

Fig. 17 illustrates an EEG signals evoking system according to another embodiment of the present invention. The EEG signal evoking system of Fig. 17 is similar to the EEG signal evoking system of Fig. 15 except that a mixer 27 is connected to the output terminals of the automatic level adjusting circuits 12a, 12b for mixing detected alpha wave signals from the right and left cerebral hemispheres and sending the mixed signals to the photic stimulus generators 5a, 5b.

According to the EEG signal evoking system shown in Fig. 17, the right and left cerebral hemispheres of the user's brain are stimulated not only by each other's brain waves but also by brain waves of their own. Thus, even if either one of the right and left cerebral hemispheres in the closed loop has a lower level of evoked response, or even if the grain of the closed loop is low at the start of brain wave evocation, stable brain waves can be evoked.

While the alpha waves are evoked in the illustrated embodiments, theta and beta waves can also be evoked in the same manner as described above by varying the passbands of the bandpass filters used. It should be noted that in order to evoke the alpha waves, the EEG signals having frequencies ranging from 7 to 20 Hz, preferably 8 to 13 Hz need to be extracted. In order to evoke the beta waves, the EEG signals having frequencies ranging from 14 to 30 Hz need to be extracted. In order to evoke the theta waves, the EEG signals having frequencies ranging from 0.5 to 7 Hz, preferably 4 to 7 Hz need to be extracted. As a signal indicative of a physiological change of the user, there may be used a magnetoencephalogram signal, an electrodermogram signal, a dermal vibration, a dermal resistance, or the like which has a certain correlation to an EEG signal. If an electro-dermogram signal, a dermal vibration, or a dermal resistance is employed, the electrodes 1a, 1b or any of other suitable means for detecting a physiological change signal are attached to suitable body parts such as wrists. any of an aural stimulus, an electric stimulus, and a vibratory stimulus may be used instead of a photic stimulus to stimulate the body of the system user. If an aural stimulus is used, then suitable means for applying such as aural stimulus is positioned near an ear of the user. If an electric or vibratory stimulus is to be fed back to the user's body, then a suitable means for applying such an electric or vibratory stimulus is attached to a suitable body part such as a wrist.

## Claims

1. A system for evoking an electroencephalogram signal from the brain of a user of the system, comprising
detecting means (1a,1b) for detecting a physiological change in a body of the user and producing a signal indicative of the detected physiological change;
an amplifier (3) connected to the detecting means (1a,1b) for amplifying the signal indicative of the detected physiological change and producing an amplified signal;
filtering means (4) for filtering the amplified signal; and
stimulating means (5) for converting the frequency signal extracted by the filtering means (4) to a stimulative signal and for applying the stimulative signal to a user; characterised by:
the filtering means (4) extracting an electroencephalogram signal to be evoked from the signal produced by the detecting means; and
the stimulating means (5) generating a stimulative signal at a frequency which is in synchronism with the signal extracted by the filtering means.

2. A system according to claim 1, wherein the filtering means (4) comprises a bandpass filter.

3. A system according to claim 2, wherein the bandpass filter has a variable passband with a central frequency; and including
filter frequency control means (6) for analysing the frequency of the signal produced by the detecting means, selecting a peak frequency value of the analyzed frequency in a desired frequency range of the electroencephalogram signal and setting the central frequency of the passband of the bandpass filter to the selected frequency.

4. A system according to claim 1, wherein the filtering means (4) comprises a bandpass filter, and the bandpass filter is connected to the output of the amplifier (3) for extracting the electroencephalogram signal from the amplified signal produced by the amplifier (3), the stimulative signal having a variant amplitude.

5. A system according to claim 3, wherein the filter frequency control means (6) is further connected to the output of the amplifier (3).

6. A system according to claim 3 or claim 5, wherein the filter frequency control means (6) produces a clock signal having a frequency corresponding to the amplified output, and the passband of the bandpass filter (4) is varied in response to the clock signal.

7. A system according to claim 6 or claim 6, wherein the filter frequency control means (6) analyses the amplified signal by way of a fast Fourier transform.

8. A system according to any of claims 1 to 7, further comprising level adjusting means (12) for adjusting the amplitude of the frequency signal to a predetermined level and outputing a level-adjusted frequency signal.

9. A system according to claim 8, further comprising means (13,14) for detecting the level of the evoked electroencephalogram signal from the amplitude of the frequency signal extracted by the bandpass filter and producing an audible signal corresponding to the level of the detected evoked electroencephalogram signal.

10. A system according to claim 8 or claim 9, wherein the level adjusting means (12) comprises an automatic gain control circuit, a limiter or a clipper.

11. A system according to claim 2, further comprising a phase lock signal generator (16) for generating a signal having a constant level in phase with the signal produced by the detecting means.

12. A system according to claim 11, further comprising a second bandpass filter (17) for removing noise from the signal produced by the detecting means.

13. A system according to claim 2, further comprising pulse width modulating means (26) for generating a signal of a constant level which has been pulse-width-modulated by the frequency signal from the first band pass filter (15); and, a second bandpass filter (17) for extracting a frequency signal corresponding to an electroencephalogram signal to be evoked from the signal produced by the pulse width modulator means.

14. A system according to any of claims 1 to 13, wherein the frequency signal represents a theta wave having a frequency ranging from 0.5 to 7 Hz.

15. A system according to any of claims 1 to 13, wherein the frequency signal represents an alpha wave having a frequency ranging from 7 to 20 Hz.

16. A system according to any of the preceding claims, wherein the detecting means comprises a pair of electrodes (1a,1b) attachable to the head of the user.

17. A system according to any of claims 1 to 13, wherein the stimulating means (5) comprises a light emitting diode and a driver for energizing the light emitting diode.

18. A system according to claim 17, wherein the stimulating means provides a photic stimulus to the user in synchronism with changes in the amplitude of the stimulative signal.

19. A signal according to any of claims 1 to 13, wherein the stimulating means applies an aural stimulus to the user.

20. A system according to any of claims 1 to 13, wherein the stimulating means applies an electric stimulus to the user.

21. A system according to any of claims 1 to 13, wherein the stimulating means applies a vibratory stimulus to the user.

22. A system according to any of claims 1 to 13, wherein the detecting means and the stimulating means are incorporated in a headband attachable to the user.

23. A system according to claim 1, wherein
the detecting means (1a,1b) comprises left and right detecting means for detecting physiological changes in the body of the user relative to the right and left cerebral hemispheres from a plurality of areas on the body of the user and producing signals indicative of the detected physiological changes, respectively;
the system further comprises a plurality of bandpass filters (4) for extracting the desired electroencephalogram signals from the signal produced by the left and right detecting means respectively, and level adjusting means (12) for adjusting the amplitude levels of output signals from the bandpass filters (4) to respective predetermined levels; and
the stimulative means (5) is adapted to generate stimulative signals from the output signals from the level adjusting means respectively, applying the stimulative signal based on the detected physiological change signal relative to the right hemisphere back to the left cerebral hemisphere, and apply the stimulative signal based on the detected physiological change signal relative to the left cerebral hemisphere back to the right cerebral hemisphere.

24. A system according to claim 23, further comprising a mixer (27) connected to the output terminals of the level adjusting means (12) and to the stimulative means (5).

## Patentansprüche

1. System zur Evozierung eines Elektroenzephalogrammsignals von dem Gehirn eines Anwenders des Systems, das umfaßt:
eine Erfassungseinrichtung (1a, 1b), die eine physiologische Veränderung im Körper eines Anwenders ermittelt und ein Signal erzeugt, das die ermittelte physiologische Veränderung anzeigt;
einen mit der Erfassungseinrichtung (1a, 1b) verbundenen Verstärker (3), der das die ermittelte physiologische Veränderung anzeigende Signal verstärkt und ein verstärktes Signal erzeugt;
eine Filterungseinrichtung (4), die das verstärkte Signal filtert, und
eine Stimulationseinrichtung (5), die das von der Filterungseinrichtung (4) extrahierte Frequenzsignal in ein stimulierendes Signal umwandelt und das stimulierende Signal an einen Anwender anlegt;
gekennzeichnet durch:
die Filterungseinrichtung (4), die ein Elektroenzephalogrammsignal extrahiert, das von dem durch die Erfassungseinrichtung erzeugten Signal zu evozieren ist, und
die Stimulationseinrichtung (5), die ein stimulierendes Signal mit einer Frequenz erzeugt, die sich in Synchronismus mit dem von der Filterungseinrichtung extrahierten Signal befindet.

2. System nach Anspruch 1, bei dem die Filterungseinrichtung (4) ein Bandpaßfilter umfaßt.

3. System nach Anspruch 2, bei dem das Bandpaßfilter ein veränderliches Paßband mit einer Mittenfrequenz besitzt, und umfassend
eine Filterfrequenz-Steuereinrichtung (6), die die Frequenz des von der Erfassungseinrichtung erzeugten Signals analysiert, einen Spitzenfrequenzwert der analysierten Frequenz in einem gewünschten Frequenzbereich des Elektroenzephalogrammsignals auswählt und die Mittenfrequenz des Paßbandes des Bandpaßfilters auf die ausgewählte Frequenz einstellt.

4. System nach Anspruch 1, bei dem die Filterungseinrichtung (4) ein Bandpaßfilter umfaßt und das Bandpaßfilter mit dem Ausgang des Verstärkers (3) verbunden ist, um das Elektroenzephalogrammsignal aus dem vom Verstärker (3) erzeugten verstärkten Signal zu extrahieren, wobei das stimulierende Signal eine abweichende Amplitude aufweist.

5. System nach Anspruch 3, bei dem die Filterfrequenz-Steuereinrichtung (6) weiter mit dem Ausgang des Verstärkers (3) verbunden ist.

6. System nach Anspruch 3 oder 5, bei dem die Filterfrequenz-Steuereinrichtung (6) ein Taktsignal mit einer Frequenz erzeugt, die dem verstärkten Ausgang entspricht, und das Paßband des Bandpaßfilters (4) als Reaktion auf das Taktsignal verändert wird.

7. System nach Anspruch 3 oder 6, bei dem die Filterfrequenz-Steuereinrichtung (6) das verstärkte Signal auf dem Wege einer schnellen Fourier-Transformation analysiert.

8. System nach einem der Ansprüche 1 bis 7, weiter umfassend eine Pegeleinstelleinrichtung (12), die die Amplitude des Frequenzsignals auf einen vorbestimmten Pegel einstellt und ein gepegeltes Frequenzsignal ausgibt.

9. System nach Anspruch 8, weiter umfassend eine Einrichtung (13, 14) zum Ermitteln des Pegels des evozierten Elektroenzephalogrammsignals aus der Amplitude des von dem Bandpaßfilter extrahierten Frequensignals und zum Erzeugen eines hörbaren Signals, das dem Pegel des ermittelten evozierten Elektroenzephalogrammsignals entspricht.

10. System nach Anspruch 8 oder 9, bei dem die Pegeleinstelleinrichtung (12) eine automatische Verstärkungsregelungsschaltung, einen Begrenzer oder einen Clipper umfaßt.

11. System nach Anspruch 2, weiter umfassend einen phasenverriegelten Signalgenerator (16), der ein Signal mit einem konstanten Pegel in Phase mit dem von der Erfassungseinrichtung erzeugten Signal erzeugt.

12. System nach Anspruch 11, weiter umfassend ein zweites Bandpaßfilter (17), um Rauschen von dem durch die Erfassungseinrichtung erzeugten Signal zu entfernen.

13. System nach Anspruch 2, weiter umfassend eine Pulsbreitenmodulationseinrichtung (26), um ein Signal eines konstanten Pegels zu erzeugen, das durch das Frequenzsignal von dem ersten Bandpaßfilter (15) pulsbreitenmoduliert worden ist; und ein zweites Bandpaßfilter (17), um ein Frequenzsignal, das einem zu evozierenden Elektroenzephalogrammsignal entspricht, aus dem von der Pulsbreitenmodulationseinrichtung erzeugten Signal zu extrahieren.

14. System nach einem der Ansprüche 1 bis 13, bei dem das Frequenzsignal eine Thetawelle mit einer Frequenz im Bereich von 0.5 bis 7 Hz darstellt.

15. System nach einem der Ansprüche 1 bis 13, bei dem das Frequenzsignal eine Alphawelle mit einer Frequenz im Bereich von 7 bis 20 Hz darstellt.

16. System nach einem der vorangehenden Ansprüche, bei dem die Erfassungseinrichtung ein am Kopf des Anwenders anzubringendes Elektrodenpaar (1a, 1b) umfaßt.

17. System nach einem der Ansprüche 1 bis 13, bei dem die Stimulationseinrichtung (5) eine lichtemittierende Diode und einen Treiber zum Erregen der lichtemittierenden Diode umfaßt.

18. System nach Anspruch 17, bei dem die Stimulationseinrichtung dem Anwender einen optischen Stimulus in Synchronismus mit Änderungen in der Amplitude des stimulierenden Signals liefert.

19. System nach einem der Ansprüche 1 bis 13, bei dem die Stimulationseinrichtung einen akustischen Stimulus an den Anwender anlegt.

20. System nach einem der Ansprüche 1 bis 13, bei dem die Stimulationseinrichtung einen elektrischen Stimulus an den Anwender anlegt.

21. System nach einem der Ansprüche 1 bis 13, bei dem die Stimulationseinrichtung einen vibrierenden Stimulus an den Anwender anlegt.

22. System nach einem der Ansprüche 1 bis 13, bei dem die Erfassungseinrichtung und die Stimulationseinrichtung in einem am Anwender anzubringenden Kopfband vereinigt sind.

23. System nach Anspruch 1, bei dem
die Erfassungseinrichtung (1a, 1b) eine linke und rechte Erfassungseinrichtung umfaßt, um physiologische Veränderungen im Körper des Anwenders in bezug auf die rechte und linke Großhirnhälfte von einer Mehrzahl von Bereichen am Körper des Anwenders zu erfassen bzw. Signale zu erzeugen, die die erfaßten physiologischen Veränderungen anzeigen;
das System weiter eine Mehrzahl von Bandpaßfiltern (4), um die gewünschten Elektroenzephalogrammsignale aus dem von der linken bzw. rechten Erfassungseinrichtung erzeugten Signal zu extrahieren, sowie eine Pegeleinstelleinrichtung (12) umfaßt, um die Amplitudenpegel der Ausgangssignale von den Bandpaßfiltern (4) auf jeweilige vorbestimmte Pegel einzustellen, und
die Stimulationseinrichtung (5) eingerichtet ist, um stimulierende Signale aus den Ausgangssignalen von der Pegeleinstelleinrichtung zu erzeugen, bzw. das stimulierende Signal, das auf dem erfaßten physiologischen Veränderungssignal in bezug auf die rechte Großhirnhälfte basiert, zurück an die linke Großhirnhälfte anzulegen, und das stimulierende Signal, das auf dem erfaßten physiologischen Veränderungssignal in bezug auf die linke Großhirnhälfte basiert, zurück an die rechte Großhirnhälfte anzulegen.

24. System nach Anspruch 23, weiter umfassend einen Mischer (27), der mit den Ausgängen der Pegeleinstelleinrichtung (12) und mit der Stimulationseinrichtung (5) verbunden ist.

## Revendications

1. Système permettant d'évoquer un signal électroencéphalographique à partir du cerveau d'un utilisateur du système, comprenant :
un moyen de détection (1a, 1b) pour détecter une modification physiologique dans le corps de l'utilisateur et pour produire un signal indicatif de la modification physiologique détectée ;
un amplificateur (3) connecté au moyen de détection (1a, 1b) pour amplifier le signal indicatif de la modification physiologique détectée et pour produire un signal amplifié ;
un moyen de filtrage (4) pour filtrer le signal amplifié ; et
un moyen de stimulation (5) pour convertir le signal de fréquence extrait par le moyen de filtrage (4) en un signal de stimulation et pour appliquer le signal de stimulation à un utilisateur,
caractérisé en ce que :
le moyen de filtrage (4) extrait un signal électroencéphalographique qui doit être évoqué à partir du signal produit par le moyen de détection ; et
le moyen de stimulation (5) génère un signal de stimulation à une fréquence qui est en synchronisation avec le signal extrait par le moyen de filtrage.

2. Système selon la revendication 1, dans lequel le moyen de filtrage (4) comprend un filtre passe-bande.

3. Système selon la revendication 2, dans lequel le filtre passe-bande présente une bande passante variable comportant une fréquence centrale ; et incluant :
un moyen de commande de fréquence de filtre (6) pour analyser la fréquence du signal produit par le moyen de détection (6), pour sélectionner une valeur de fréquence de pic de la fréquence analysée dans une plage de fréquences souhaitée du signal électroencéphalographique et pour établir la fréquence centrale de la bande passante du filtre passe-bande à la fréquence sélectionnée.

4. Système selon la revendication 1, dans lequel le moyen de filtrage (4) comprend un filtre passe-bande et le filtre passe-bande est connecté à la sortie de l'amplificateur (3) pour extraire le signal électroencéphalographique du signal amplifié produit par l'amplificateur (3), le signal de stimulation présentant une amplitude variable.

5. Système selon la revendication 3, dans lequel le moyen de commande de fréquence de filtre (6) est en outre connecté à la sortie de l'amplificateur (3).

6. Système selon la revendication 3 ou 5, dans lequel le moyen de commande de fréquence de filtre (6) produit un signal d'horloge présentant une fréquence correspondant à la sortie amplifiée et la bande passante du filtre passe-bande (4) est amenée à varier en réponse au signal d'horloge.

7. Système selon la revendication 6, dans lequel le moyen de commande de fréquence de filtre (6) analyse le signal amplifié au moyen d'une transformation de Fourier rapide.

8. Système selon l'une quelconque des revendications 1 à 7, comprenant en outre un moyen de réglage de niveau (12) pour régler l'amplitude du signal de fréquence à un niveau prédéterminé et pour émettre en sortie un signal de fréquence à un niveau réglé.

9. Système selon la revendication 8, comprenant en outre un moyen (13, 14) pour détecter le niveau du signal électroencéphalographique évoqué à partir de l'amplitude du signal de fréquence extrait par le filtre passe-bande et pour produire un signal audible correspondant au niveau du signal électroencéphalographique évoqué détecté.

10. Système selon la revendication 8 ou 9, dans lequel le moyen de réglage de niveau (12) comprend un circuit de commande de gain automatique, un limiteur ou un écrêteur.

11. Système selon la revendication 2, comprenant en outre un générateur de signal de verrouillage de phase (16) pour générer un signal présentant un niveau constant en phase avec le signal produit par le moyen de détection.

12. Système selon la revendication 11, comprenant en outre un second filtre passe-bande (17) pour supprimer le bruit du signal produit par le moyen de détection.

13. Système selon la revendication 2, comprenant en outre un moyen de modulation de largeur d'impulsion (26) pour générer un signal d'un niveau constant qui a été modulé en largeur d'impulsion par le signal de fréquence provenant du premier filtre passe-bande (15) ; et un second filtre passe-bande (17) pour extraire un signal de fréquence correspondant à un signal électroencéphalographique qui doit être évoqué du signal produit par le moyen de modulateur de largeur d'impulsion.

14. Système selon l'une quelconque des revendications 1 à 13, dans lequel le signal de fréquence représente une onde thêta présentant une fréquence s'inscrivant dans une plage de 0,5 à 7 Hz.

15. Système selon l'une quelconque des revendications 1 à 13, dans lequel le signal de fréquence représente une onde alpha présentant une fréquence s'inscrivant dans une plage de 7 à 20 Hz.

16. Système selon l'une quelconque des revendications précédentes, dans lequel le moyen de détection comprend une paire d'électrodes (1a, 1b) qui peuvent être fixées à la tête de l'utilisateur.

17. Système selon l'une quelconque des revendications 1 a 13, dans lequel le moyen de stimulation (5) comprend une diode émettrice de lumière et un dispositif de pilotage pour exciter la diode émettrice de lumière.

18. Système selon la revendication 17, dans lequel le moyen de stimulation produit un stimulus photique pour l'utilisateur en synchronisation avec des variations de l'amplitude du signal de stimulation.

19. Signal selon l'une quelconque des revendications 1 à 13, dans lequel le moyen de stimulation applique un stimulus auditif à l'utilisateur.

20. Système selon l'une quelconque des revendications 1 à 13, dans lequel le moyen de stimulation applique un stimulus électrique à l'utilisateur.

21. Système selon l'une quelconque des revendications 1 à 13, dans lequel le moyen de stimulation applique un stimulus vibrant à l'utilisateur.

22. Système selon l'une quelconque des revendications 1 à 13, dans lequel le moyen de détection et le moyen de stimulation sont incorporés dans un bandeau qui peut être fixé sur l'utilisateur.

23. Système selon la revendication 1, dans lequel :
le moyen de détection (1a, 1b) comprend des moyens de détection gauche et droit pour détecter des modifications physiologiques dans le corps de l'utilisateur concernant les hémisphère cérébraux droit et gauche à partir d'une pluralité de zones sur le corps de l'utilisateur et pour produire des signaux indicatifs des modifications physiologiques détectées ;
le système comprend en outre une pluralité de filtres passe-bande (4) pour extraire les signaux électroencéphalographiques souhaités du signal respectivement produit par les moyens de détection gauche et droit et un moyen de réglage de niveau (12) pour régler les niveaux d'amplitude de signaux de sortie provenant des filtres passe-bande (4) à des niveaux prédéterminés respectifs ; et
le moyen de stimulation (5) est conçu pour générer des signaux de stimulation à partir des signaux de sortie provenant respectivement des moyens de réglage de niveau, en appliquant le signal de stimulation sur la base du signal de modification physiologique détecté concernant l'hémisphère droit en retour sur l'hémisphère cérébral gauche et en appliquant le signal de stimulation sur la base du signal de modification physiologique détecté concernant l'hémisphère cérébral gauche en retour sur l'hémisphère cérébral droit.

24. Système selon la revendication 23, comprenant en outre un mélangeur (27) connecté aux bornes de sortie du moyen de réglage de niveau (12) et au moyen de stimulation (5).
